# EUROPEAN PATENT APPLICATION

(11) **EP 3 701 801 A1**
(43) Date of publication of application: **02.09.2020**
(21) Application number: 18871325.9
(22) Date of filing: 14.09.2018
(51) Int. Cl.: A23G 3/34, A23L 5/41

(54) **PYRROLOQUINOLINE-QUINONE-CONTAINING GUMMY CANDY AND METHOD FOR PRODUCING SAME**

(30) Priority: 25.10.2017 JP 2017205929
(71) Applicant: Mitsubishi Gas Chemical Company, Inc., Tokyo 100-8324 (JP)
(72) Inventor: IKEMOTO, Kazuto, Niigata-shi Niigata 950-3112 (JP)
(74) Representative: Müller-Boré & Partner Patentanwälte PartG mbB
(86) International application number: PCT/JP2018/034138
(87) International publication number: WO 2019/082550

(57) **Abstract**

Provided is a gummy candy comprising a fat and oil, a hydrophobic emulsifier, pyrroloquinoline quinone or a salt thereof, and gelatin.

## Description

### Technical Field

The present invention relates to a pyrroloquinoline quinone-containing gummy candy and a method for producing the same.

### Background Art

Gummy candies are confectionery that is composed mainly of carbohydrates and gelatin and has elastic texture, and are being loved by people all over the world. As a health trend has been improved in recent years, the emphasis has also been placed on functionality. Therefore, gummy candies supplemented with nutrient components or functional components are popular mainly in Europe and the United States.

Pyrroloquinoline quinone (hereinafter, referred to as PQQ) is known as a microbial coenzyme. Its disodium salt having water solubility is used in foods and known to have many functions such as brain function activation, mitochondrial activation, and cell growth promotion.

It is known that a salt of pyrroloquinoline quinone forms a fibrous structure by mixing its aqueous solution with a reducing agent at room temperature, and is eventually gelled (Patent Literature 1). A gel comprising pyrroloquinoline quinone is also described in Patent Literature 2. Patent Literature 3 describes a quinone compound and takes water-insoluble ubidecarenone as an example.

### Citation List

### Patent Literature

Patent Literature 1: International Publication No. WO 2012/020767
Patent Literature 2: Japanese Patent Laid-Open No. 2013-237644
Patent Literature 3: International Publication No. WO 2005/035477
Patent Literature 4: Japanese Patent Laid-Open No. 2015-010085

### Summary of Invention

### Technical Problem

Pyrroloquinoline quinone has a red color and has the disadvantage that the color is transferred into the mouth at the time of eating. Such a disadvantage is prominent, particularly, when pyrroloquinoline quinone is used in foods, such as gummy candies, which are retained in the oral cavity for a long time. Hence, pyrroloquinoline quinone needs to secure stability so as to prevent color transfer into the oral cavity when gummy candies are supplemented therewith.

Texture characteristic of gummy candies is largely attributed to a gelling agent. A problem of gelatin used as a gelling agent is the discoloration of PQQ into an orange color. However, in order to apply PQQ to gummy candies, a method for suppressing discoloration and enhancing stability as gummy candies has not yet been known.

An object of the present invention is to provide a stable gummy candy in which discoloration specific for pyrroloquinoline quinone is suppressed.

### Solution to Problem

The present inventors have conducted diligent studies to attain the object, and consequently completed the present invention by finding that the object can be attained by supplementation with a hydrophobic emulsifier and a fat and oil.

Specifically, the present invention encompasses the following aspects of the invention.
[1] A gummy candy comprising:
   a fat and oil;
   a hydrophobic emulsifier;
   pyrroloquinoline quinone or a salt thereof; and gelatin.
[2] The gummy candy according to [1], wherein the pyrroloquinoline quinone or the salt thereof is colloidized in the fat and oil in the presence of the hydrophobic emulsifier.
[3] The gummy candy according to [1] or [2], wherein the salt of pyrroloquinoline quinone is pyrroloquinoline quinone disodium salt.
[4] The gummy candy according to any of [1] to [3], wherein the hydrophobic emulsifier is at least one selected from the group consisting of sucrose fatty acid ester, glycerin fatty acid ester, glycerin-condensed ricinoleic acid ester, and sorbitan fatty acid ester.
[5] The gummy candy according to any of [1] to [4], further comprising a sweetener.
[6] A method for producing a gummy candy according to any of [1] to [5], comprising the step of colloidizing pyrroloquinoline quinone or a salt thereof in a fat and oil in the presence of a hydrophobic emulsifier.
[7] The method according to [6], comprising the step of mixing the colloidized pyrroloquinoline quinone or salt thereof with gelatin.

### Advantageous Effects of Invention

Pyrroloquinoline quinone in gummy candies prepared with gelatin tends to be easily discolored as compared with the case of using other gelling agents such as agar, sodium alginate, and carrageenan. However, according to the present invention, supplementation with a fat and oil and a hydrophobic emulsifier suppresses such discoloration and enables suppression of color transfer to the tongue or the like. Thus, a stable gummy candy supplemented with pyrroloquinoline quinone can be provided. In this context, the discoloration means the discoloration of pyrroloquinoline quinone, which originally assumes a red color, into another color such as an orange color or a yellow color, and is not change in color intensity.

### Description of Embodiments

Hereinafter, the mode for carrying out the present invention (hereinafter, simply referred to as the "present embodiment") will be described in detail. However, the present invention is not limited by the present embodiments given below. Various changes or modifications can be made in the present invention without departing from the spirit of the present invention.

According to the first embodiment, the present invention provides a gummy candy comprising a fat and oil, a hydrophobic emulsifier, pyrroloquinoline quinone or a salt thereof, and gelatin.

The gummy candy is a kind of soft candy having elastic texture resulting from supplementation with a gelling agent such as gelatin.

The pyrroloquinoline quinone according to the present invention is a substance having a structure represented by the formula (1):

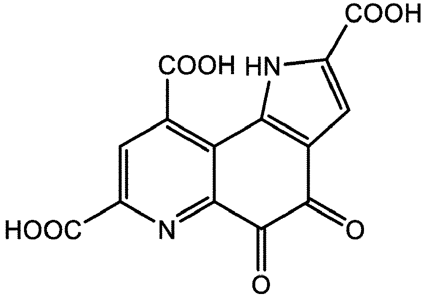

The pyrroloquinoline quinone or the salt thereof used in the present invention can be obtained as a commercially available product, and can be produced by a method known in the art.

The pyrroloquinoline quinone may be a free form. Examples of the salt of pyrroloquinoline quinone include alkali metal salts, alkaline earth metal salts and ammonium salts of pyrroloquinoline quinone. An alkali metal salt is preferred. PQQ contained in the form of a salt in the gummy candy is expected to have effects such as improvement in PQQ content, osmotic pressure adjustment, and taste adjustment.

Examples of the alkali metal salt of pyrroloquinoline quinone used in the present invention include sodium, potassium, lithium, cesium, and rubidium salts. Sodium salt or potassium salt is preferred because the salt is easily obtained.

The alkali metal salt of pyrroloquinoline quinone can be an alkali metal salt having a substitution of 1 to 3 alkali metals, and can be any of monoalkali metal salts, dialkali metal salts, and trialkali metal salts. A dialkali metal salt is preferred. Disodium salt or dipotassium salt is particularly preferred. Hydrated crystals of disodium salt are highly stable and easy to use.

The gummy candy according to the present invention comprises a fat and oil which is an ester of a fatty acid and glycerin, and a hydrophobic emulsifier. The pyrroloquinoline quinone is colloidized in the fat and oil in the presence of the hydrophobic emulsifier. A dispersion containing such colloids can be gelled with gelatin to form a gummy candy in which the discoloration of the pyrroloquinoline quinone is suppressed.

An edible fat and oil is used. Specific examples thereof include medium chain or short chain fatty acid esters. The origin of the edible fat and oil is not limited, and, for example, a plant-derived or animal-derived fat and oil can be used. Among the fats or oils, a medium chain fatty acid ester is transparent and low viscous and is therefore suitable for preparing the dispersion of pyrroloquinoline quinone. The medium chain fatty acid oil used herein means a medium chain fatty acid oil having 4 to 14 carbon atoms, preferably approximately 8 to 10 carbon atoms.

Examples of the plant oil include, but are not particularly limited to, olive oil, rice oil, salad oil, corn oil, soybean oil, rapeseed oil, cacao butter, cottonseed oil, palm oil, castor oil, sesame oil, jojoba oil, safflower oil, almond oil, avocado oil, camellia oil, cacao oil, and coconut oil. These oils are often used, particularly, in the food field, for example, without particular limitations.

Examples of the animal oil include, but are not particularly limited to, beef tallow, lard, fish oil, squalene, mink oil, turtle oil, and egg yolk oil. These oils are often used, particularly, in the food field, for example, without particular limitations.

The gelling agent used in the present invention is gelatin. Carrageenan, agar, or collagen may be adjunctively added to the gelling agent as long as gelatin is contained therein. The amount of the gelling agent added can be appropriately adjusted according to texture required for the gummy candy as a final product. The amount of the gelling agent in the gummy candy is usually within the range of, for example, 5 to 15% by weight.

A hydrophobic emulsifier can be used for dispersing an aqueous solution containing the pyrroloquinoline quinone into the fat and oil. Examples of such an emulsifier include sucrose fatty acid ester, glycerin fatty acid ester, glycerin-condensed ricinoleic acid ester, and sorbitan fatty acid ester.

The hydrophobic emulsifier comprises at least one selected from the group consisting of sucrose polyerucate, sucrose polyoleate, sucrose polystearate, sucrose polypalmitate, sucrose polymyristate, sucrose polylaurate, monoglycerin oleic acid ester, diglyceryl triisostearate, tetraglyceryl pentaoleate, decaglyceryl pentaoleate, decaglyceryl heptaoleate, decaglyceryl decaoleate, tetraglycerin-condensed ricinoleic acid ester, hexaglycerin-condensed ricinoleic acid ester, polyglycerin-condensed ricinoleic acid ester, sorbitan oleate, sorbitan dioleate, and sorbitan trioleate.

The weight ratio of the hydrophobic emulsifier to PQQ or the salt thereof (hydrophobic emulsifier/PQQ or the salt thereof) is preferably 2.5 to 500.

In order to stabilize the pyrroloquinoline quinone, the pyrroloquinoline quinone is preferably dispersed in the fat and oil. In this context, the term "dispersed" means that a homogeneous state is maintained without observed precipitation of PQQ or the salt thereof, separation between PQQ or the salt and the edible fat and oil, etc. after a lapse of 48 hours from preparation of a mixed solution of PQQ or the salt thereof with the edible fat and oil.

The dispersibility can be adjusted by the mixing ratio between PQQ and the emulsifier, the average particle size of PQQ and/or the salt thereof dispersed in the oil, the water content and/or salt content of PQQ dispersed in the oil, the type of the emulsifier, etc. The emulsifier must have HLB of 5 or less. The content of PQQ is preferably 0.01 to 20% by mass, more preferably 0.02 to 10% by mass, further preferably 0.05 to 5.0% by mass, with respect to the whole dispersion.

The dispersion is prepared such that the gummy candy comprises 0.00001 to 4% of PQQ, 0.1 to 20% of the fat and oil, and 0.0001 to 4% of the emulsifier. The gummy candy of the present invention is prepared by using 0.1 to 20% by weight of the dispersion in the total weight of the gummy candy, though the amount of the dispersion varies depending on the amount of the pyrroloquinoline quinone or the fat and oil. Too large an amount of the dispersion may cause phase separation and make it difficult to maintain the shape of the gummy candy. Furthermore, the gummy candy may become oily.

A component other than those described above can be added, if necessary, to the dispersion according to the present embodiment.

If desired colloidal particles are not formed using the emulsifier, an auxiliary can be added. Examples of such an auxiliary include, but are not particularly limited to, polyoxyethylene sorbitol tetraisostearate, polyoxyethylene sorbitol tetraoleate, polyoxyethylene sorbitol monolaurate, polyoxyethylene glyceryl triisostearate, polyoxyethylene glyceryl oleate, polyethylene glycol monolaurate, polyethylene glycol monooleate, polyethylene glycol diisostearate, polyoxyethylene oleyl ether, polyoxyethylene lauryl ether, polyoxyethylene hydrogenated castor oil, polyoxyethylene sorbitan monooleate, polyoxyethylene sorbitan monostearate, polyoxyethylene sorbitan monolaurate, polyoxyethylene sorbitan isostearate, polyoxyethylene sorbitan trioleate, polyglycerin isostearic acid ester, polyglycerin lauric acid ester and polyglycerin oleic acid ester. These auxiliaries may be used alone or used in combination of two or more thereof.

The gummy candy is usually prepared through a cooling step and a drying step, after addition of a gelatin solution, fruit juice, an acidulant, a flavor, a colorant, and the like to a concentrated sugar solution. A sweetener that can be used in foods is appropriately selected as a raw material for such a sugar solution. Examples of the food sweetener include sucrose, sorbitol, xylitol, Palatinit, glucose, fructose, isomerized sugar, aspartame, neotame, glycyrrhizin, saccharin, stevioside, rebaudioside, dulcin, alitame, trichlorosucrose, thaumatin, acesulfame potassium, and sucralose.

Fruit juice, vegetable juice, a plant extract, or the like can be arbitrarily selected as the fruit juice. The type of the acidulant, the flavor, the colorant, or the like is not particularly limited.

The gummy candy may be appropriately supplemented with functional materials such as dietary fiber, vitamins, minerals, and amino acids, a fat and oil, an emulsifier, and/or a dairy product.

In the case of using a hydrophobic emulsifier in the preparation of the gummy candy, it is considered that the pyrroloquinoline quinone and/or the salt thereof is colloidized and dispersed in the edible fat and oil. The size of the particles of the colloidized pyrroloquinoline quinone and/or salt thereof may be within the range of, for example, 2 nm to 200 µm, preferably 50 nm to 100 µm. A smaller size of the particles enhances an effect of suppressing discoloration. An oil having a small size of the particles appears to be in a solution state and enhances the homogeneity of a product.

According to the second embodiment, the present invention provides a method for producing the gummy candy, comprising the step of colloidizing pyrroloquinoline quinone or a salt thereof with a hydrophobic emulsifier in a fat and oil.

The gummy candy of the present invention is characterized by being prepared by colloidizing pyrroloquinoline quinone in a fat and oil in the presence of a hydrophobic emulsifier. For example, pyrroloquinoline quinone and/or a salt thereof, water, and an emulsifier are homogenized in an edible fat and oil. Then, the edible fat and oil solution obtained by removing water can be mixed with a solution containing gelatin, water, and a sweetener to produce the gummy candy of the present invention.

More specifically, an aqueous solution of pyrroloquinoline quinone and/or a salt thereof in a dissolved or suspended state is provided, and this aqueous solution is added to an oil supplemented with an emulsifier. After emulsification with a homomixer or the like, water is removed to form colloids dispersed in the oil (dispersion). Although not wishing to be bound by any theory, the dispersion of pyrroloquinoline quinone prepared before supplementation with a gelling agent is considered to stabilize the pyrroloquinoline quinone and suppress the discoloration of the gummy candy.

A temperature for the colloidization is preferably 40 to 100°C, more preferably 70 to 95°C. The removal of water from the dispersion can be performed by reduction in pressure using an evaporator or the like, or freeze drying. A temperature for the removal of water under reduced pressure is preferably 20 to 90°C, more preferably 40 to 80°C. In an operation that substitutes as the evaporator, water may be removed by blowing nitrogen gas to the solution after the colloidization treatment, increasing the temperature to, for example, 200°C with stirring, and keeping the temperature for a predetermined time.

A gummy candy containing a high concentration of pyrroloquinoline quinone can be obtained by dissolving a larger amount of the pyrroloquinoline quinone and/or the salt thereof in water. For such a purpose, examples of the salt that exhibits alkalinity so as to enhance the solubility of the pyrroloquinoline include NaOH, KOH, Na₂CO₃, K₂CO₃, NaHCO₃, KHCO₃, Na₃PO₃, K₃PO₃, NaHPO₃, KHPO₃, sodium citrate, potassium citrate, NaCl, KCl, CaCO₃, CaCl₂, and salts of phosphoric acid and alkali metals. These salts may be used alone or may be used in combination of two or more thereof.

Such a method may further comprise the step of mixing the colloidized pyrroloquinoline quinone or salt thereof with gelatin.

Gelatin as a gelling agent and, optionally, a sweetener are dissolved in water or a buffer solution, and the obtained solution is heated to prepare a gelling agent solution. The obtained gelling agent solution can be mixed with the dispersion to produce a gummy candy.

A temperature at which the gelling agent solution is mixed with the dispersion is not particularly limited. The mixing is preferably performed at 30 to 100°C because the mixing is difficult to attain at a low temperature.

The content of each component in the gummy candy is preferably adjusted to the following range from the viewpoint of the texture and taste, shape retention, and production aptitude of the gummy candy.

The content of the sweetener is preferably adjusted to 0.55 to 85% by weight.

The content of the gelling agent is preferably adjusted to 5 to 15% by weight.

The content of water is preferably adjusted to 10 to 30% by weight.

The sweetener may be added during the mixing of the dispersion with the gelling agent. In the case of mixing these components at the same time, it is preferred that the components should not be placed at a high temperature for a long time, for improvement in stability of PQQ.

The amount of the jelly of the present invention ingested is preferably 10 to 50 mg per day in terms of pyrroloquinoline quinone disodium trihydrate. This ingested amount is preferred because moisturization of the skin, improvement in brain functions, and improvement in lipid can be expected.

### Examples

Hereinafter, the present invention will be described in more detail with reference to Examples. However, the present invention is not limited by these Examples.

The pyrroloquinoline quinone disodium salt used was BioPQQ(Registered Trademark) manufactured by Mitsubishi Gas Chemical Co., Inc. (hereinafter, abbreviated to Na₂PQQ). This product is crystalline and is stable in a trihydrate form. Other regents used were manufactured by Wako Pure Chemical Industries, Ltd.

### Example 1

4.5 g of pyrroloquinoline quinone disodium salt was dissolved in 0.3 g of potassium carbonate and 180 mL of water to obtain an aqueous solution. Meanwhile, 16 g of polyglycerin ricinoleic acid ester (CRS-75 manufactured by Sakamoto Yakuhin Kogyo Co., Ltd.) as an emulsifier and 79.5 g of medium chain fatty acid glycerin ester (MCT, Coconard MT manufactured by Kao Corp.; caprylic/capric triglyceride) as an oil were mixed at room temperature. To the obtained solution, the aqueous solution was added, and the mixture was emulsified using a homomixer manufactured by PRIMIX Corp. This emulsification treatment elevated the temperature to 40°C. The emulsion was placed in an eggplant-shaped flask. Water was removed from the emulsion in the eggplant-shaped flask by reduction in pressure using an evaporator in which the temperature of a warm bath was set to 50°C. The removal of water from the emulsion resulted in a dispersion of colloidal particles containing pyrroloquinoline quinone. After a lapse of 48 hours, no precipitation was confirmed. A red transparent solution was obtained as the dispersion. The particle size was measured by the dynamic light scattering method using ELS-Z manufactured by Otsuka Electronics Co., Ltd. The particle size was 88 nm. In the dispersion containing colloidized particles having a such a small size, the amount of the emulsifier in the coating is large with respect to the weight of the pyrroloquinoline quinone particles. Hence, an effect of suppressing discoloration is high.

80 ml of a beverage containing 10% apple juice (Asahi Soft Drinks Co., Ltd., Bireley's Apple), 10 g of a gelatin powder (Morinaga Cook Gelatin manufactured by Morinaga & Co., Ltd.), and 10 g of table sugar were mixed. This mixture was heated until boiled in a microwave oven, and thereby dissolved.

To this gelatin-containing solution, the dispersion was added at 10% by weight at 70°C to prepare a solution finally containing 0.45% by weight of the pyrroloquinoline quinone, 7.9% by weight of the fat and oil, and 1.6% by weight of the emulsifier. The solution was further boiled in a microwave oven and then brought back to room temperature for solidification. A red apple-flavored gummy candy was obtained (Example 1). This gummy candy was dissolved by preservation at 70°C, preserved in this state for 1 day, and then brought back to room temperature again for solidification. The color was not changed. This gummy candy was stable. The effect of suppressing discoloration by the fat and oil was similarly confirmed after preservation at 120°C for 1 day. On the other hand, the effect of suppressing discoloration by the fat and oil was not confirmed when carrageenan, agar, or sodium alginate was used instead of gelatin.

### Comparative Example 1

An aqueous solution containing 4.5% by weight of pyrroloquinoline quinone disodium salt was prepared. A gummy candy was prepared in the same way as in Example 1 except that the gummy candy was not supplemented with the fat and oil. The gummy candy was obtained, but discolored yellow, when preserved at 70°C for 1 day and then solidified.

### Examples 2 and 3

An experiment was conducted using varying amounts of the dispersion added. Each gummy candy was prepared in the same way as in Example 1 except that the amount of the dispersion added was decreased, and the amount of water was increased by that amount. The results are shown in Table 1.

**[Table 1]**

| | Amount of dispersion added (% by weight) | Amounts of pyrroloquinoline quinone, fat and oil and emulsifier contained in gummy candy (% by weight) | Color of gummy candy | Texture |
|---|---|---|---|---|
| Example 2 | 5 | Pyrroloquinoline quinone disodium salt 0.225% | Red color | Favorable mouth feel |
| | | Fat and oil 3.975% | | |
| | | Emulsifier 0.8% | | |
| Example 3 | 1 | Pyrroloquinoline quinone disodium salt 0.045% | Light red color | Favorable mouth feel |
| | | Fat and oil 0.795% | | |
| | | Emulsifier 0.16% | | |

In both Examples, the red color of pyrroloquinoline quinone was maintained. Also, the taste was good.

### Reference Example 1 and Comparative Examples 2 and 3

A gummy candy was prepared in the same way as in Example 1 except that the amount of the fat and oil added was increased (Reference Example 1). A gummy candy was prepared by directly adding pyrroloquinoline quinone disodium salt instead of the dispersion to the edible fat and oil without the use of the emulsifier (Comparative Example 2). A test was conducted using the aqueous solution of Comparative Example 1 (Comparative Example 3) .

**[Table 2]**

| | Amount of dispersion added (% by weight) | Amounts of pyrroloquinoline quinone, fat and oil and emulsifier contained in gummy candy (% by weight) | Color of gummy candy | Texture |
|---|---|---|---|---|
| Reference Example 1 | 50 | Pyrroloquinoline quinone disodium salt 2.25% | Oil separated | Oily |
| | | Fat and oil 39.75% | | |
| | | Emulsifier 8% | | |
| Comparative Example 2 | 0 (Suspension: 1 (4.5% by weight of PQQ disodium salt and balance of edible fat and oil) | Pyrroloquinoline quinone disodium salt 0.45% | Yellow color | Favorable mouth feel |
| | | Fat and oil 9.955% | | |
| | | Emulsifier 0% | | |
| Comparative Example 3 | 0 (Aqueous solution: 1 (4.5% by weight of PQQ disodium salt and balance of water) | Pyrroloquinoline quinone disodium salt 0.45% | Yellow color | Favorable mouth feel |
| | | Fat and oil 0% | | |
| | | Emulsifier 0% | | |

The gummy candy of Reference Example 1 retained a red color, but offered poor mouth feel due to the addition of the fat and oil in a large amount.

In Comparative Example 2 in which the fat and oil was used alone, pyrroloquinoline quinone having a red color was discolored yellow. Mere suspension in the oil containing no emulsifier was free from a stabilizing effect.

In Comparative Example 3 in which neither the fat and oil nor the emulsifier was added, pyrroloquinoline quinone having a red color was discolored. The pyrroloquinoline quinone was altered by only the blending process.

### Examples 4 to 10 Dispersions differing in PQQ concentration

Each gummy candy was produced in the same way as in Example 1 except that the amount of the dispersion added prepared in Example 1 was changed. The concentrations of the added dispersion are shown in Tables 3 and 4.

**[Table 3]**

| | PQQ concentration in dispersion (% by weight) | Composition of dispersion | Amounts of pyrroloquinoline quinone, fat/oil and emulsifier contained in gummy candy (% by weight) | Amount of dispersion added to gummy candy (% by weight) | Color and texture of gummy candy |
|---|---|---|---|---|---|
| Example 4 | 1 | Na₂PQQ 1 wt% | Pyrroloquinoline quinone disodium salt 0.1% | 10 | Red color, favorable |
| | | K₂CO₃ 0.25 wt% | | | |
| | | CRS-75 10 wt% | Fat and oil 8.875% | | |
| | | Balance, edible fat and oil | Emulsifier 1% | | |
| Example 5 | 3 | Na₂PQQ 3 wt% | Pyrroloquinoline quinone disodium salt 0.3% | 10 | Red color, favorable |
| | | K₂CO₃ 0.6 wt% | | | |
| | | CRS-75 15 wt% | Fat and oil 8.14% | | |
| | | Balance, edible fat and oil | Emulsifier 1.5 % | | |
| Example 6 | 6 | Na₂PQQ 6 wt% | Pyrroloquinoline quinone disodium salt 0.6% | 10 | Red color, favorable |
| | | K₂CO₃ 1.2 wt% | | | |
| | | CRS-75 15 wt% | Fat and oil 7.78% | | |
| | | Balance, edible fat and oil | Emulsifier 1.5% | | |
| Example 7 | 10 | Na₂PQQ 10 wt% | Pyrroloquinoline quinone disodium salt 1% | 10 | Dark red color, favorable |
| | | CRS-75 20 wt% | | | |
| | | Balance, edible fat and oil | Fat and oil 7% | | |
| | | | Emulsifier 2% | | |

**[Table 4]**

| | PQQ concentration in dispersion (% by weight) | Composition of dispersion | Amounts of pyrroloquinoline quinone, fat and oil and emulsifier contained in gummy candy (% by weight) | Amount of dispersion added to gummy candy (% by weight) | Color and texture of gummy candy |
|---|---|---|---|---|---|
| Example 8 | 20 | Na₂PQQ 20 wt% | Pyrroloquinoline quinone disodium salt 2% | 10 | Dark red color, favorable |
| | | CRS-75 40 wt% | | | |
| | | Balance, edible | Fat and oil 4% | | |
| | | fat and oil | Emulsifier 4% | | |
| Example 9 | 6 | Na₂PQQ 6 wt% | Pyrroloquinoline quinone disodium salt 0.6% | 1 | Red color, favorable |
| | | K₂CO₃ 1.2 wt% | | | |
| | | CRS-75 15 wt% | Fat and oil 7.78% | | |
| | | Balance, edible fat and oil | Emulsifier 0.15% | | |
| Example 10 | 20 | Na₂PQQ 20 wt% | Pyrroloquinoline quinone disodium salt 0.2% | 1 | Dark red color, favorable |
| | | CRS-75 40 wt% | | | |
| | | Balance, edible fat and oil | Fat and oil 0.4% | | |
| | | | Emulsifier 0.4% | | |

Gummy candies were formed even when the concentrations of the pyrroloquinoline quinone and the fat and oil were changed. All the gummy candies had a red color, which was not changed into any other color. The taste was also good. A higher concentration of the pyrroloquinoline quinone disodium salt in the dispersion was able to form a darker red gummy candy.

### (Study on color transfer to tongue)

3.5 g of the gummy candy of Example 1 was put in the mouth, chewed for 1 minute, and then swallowed. Color transfer to the tongue was absent. As a result of similarly testing the gummy candy of Comparative Example 1, the tongue was turned red and then purple.

The gummy candy of the present invention is free from color transfer to the tongue. Thus, the gummy candy was excellent without problems associated with discoloration or color transfer.

### (Study on production method)

1 g of a gelling agent was added to 9.5 mL of water and dissolved by warming at 121°C for 30 minutes in an autoclave sterilization apparatus. After the temperature of the solution reached 70°C, the solution was mixed with 30 mg of pyrroloquinoline quinone disodium salt and 0.5 ml of an edible oil (The Nisshin OilliO Group, Ltd., ODO) to prepare a homogenous solution. The solution was brought back to room temperature to prepare a gummy candy (Comparative Example 4). After a lapse of 1 day or more, its look was observed.

**[Table 5]**

| | Color |
|---|---|
| Comparative Example 4 | Discolored orange |

### Industrial Applicability

The present invention relates to the field of gummy candies and is a useful technique.

## Claims

1. A gummy candy comprising:
a fat and oil;
a hydrophobic emulsifier;
pyrroloquinoline quinone or a salt thereof; and
gelatin.

2. The gummy candy according to claim 1, wherein the pyrroloquinoline quinone or the salt thereof is colloidized in the fat and oil in the presence of the hydrophobic emulsifier.

3. The gummy candy according to claim 1 or 2, wherein the salt of pyrroloquinoline quinone is pyrroloquinoline quinone disodium salt.

4. The gummy candy according to any one of claims 1 to 3, wherein the hydrophobic emulsifier is at least one selected from the group consisting of sucrose fatty acid ester, glycerin fatty acid ester, glycerin-condensed ricinoleic acid ester, and sorbitan fatty acid ester.

5. The gummy candy according to any one of claims 1 to 4, further comprising a sweetener.

6. A method for producing a gummy candy according to any one of claims 1 to 5, comprising the step of colloidizing pyrroloquinoline quinone or a salt thereof in a fat and oil in the presence of a hydrophobic emulsifier.

7. The method according to claim 6, comprising the step of mixing the colloidized pyrroloquinoline quinone or salt thereof with gelatin.
